(19) Europäisches Patentamt
European Patent Office
Office européen des brevets

(11) Publication number: **0 504 419 A1**

(12) # EUROPEAN PATENT APPLICATION published in accordance with Art. 158(3) EPC

(21) Application number: **91917065.4**

(22) Date of filing: **04.10.91**

(86) International application number: **PCT/JP91/01352**

(87) International publication number: **WO 92/06074 (16.04.92 92/09)**

(51) Int. Cl.5: **C07C 245/08**, C07C 255/65, C07C 309/46, C07C 331/08, C08F 20/60, C08F 220/28, C09B 69/10, C09K 9/02, G03C 1/73, G03F 7/00

(30) Priority: **05.10.90 JP 268059/90**
**06.11.90 JP 300898/90**
**07.11.90 JP 301750/90**

(43) Date of publication of application: **23.09.92 Bulletin 92/39**

(84) Designated Contracting States: **DE FR GB IT NL**

(71) Applicant: **SEIKO EPSON CORPORATION**
**4-1, Nishishinjuku 2-chome**
**Shinjuku-ku Tokyo 160(JP)**

(72) Inventor: **TANIGUCHI, Makoto**
**Seiko Epson Corporation, 3-5, Yamato 3-chome**
**Suwa-shi, Nagano 392(JP)**
Inventor: **UEHARA, Masamitsu**
**Seiko Epson Corporation, 3-5, Yamato 3-chome**
**Suwa-shi, Nagano 392(JP)**
Inventor: **KITAMURA, Kazuhiko**
**Seiko Epson Corporation, 3-5, Yamato 3-chome**
**Suwa-shi, Nagano 392(JP)**
Inventor: **ISHII, Isao**
**Seiko Epson Corporation, 3-5, Yamato 3-chome**
**Suwa-shi, Nagano 392(JP)**
Inventor: **MIZUMA, Isao**
**Seiko Epson Corporation, 3-5, Yamato 3-chome**
**Suwa-shi, Nagano 392(JP)**
Inventor: **OYAMA, Noriko**
**Seiko Epson Corporation, 3-5, Yamato 3-chome**
**Suwa-shi, Nagano 392(JP)**

(74) Representative: **Lewin, John Harvey**
**Elkington and Fife Prospect House 8**
**Pembroke Road**
**Sevenoaks, Kent TN13 1XR(GB)**

(54) **PHOTOSENSITIVE AZO COMPOUND AND IMAGE FORMING DEVICE UTILIZING THE SAME.**

(57) A photosensitive azo compound represented by general formula (I), wherein $R^1$ and $R^2$ may be the same or different from each other and each represents halogen, halogenated $C_1$ to $C_6$ alkyl, $-NO_2$, -SC≡N, -C≡N, $-SO_3H$, $-C_nF_{2n}COOF$, $-C_nF_{2n}COF$, $-C_nF_{2n}COOC_mF_{2m+1}$, wherein n and m each represents an integer of 1 to 6, $-SO_2NH_2$, $-SO_2NHCF_3$ or $-SO_2NHCH_3$; and $R^3$ represents hydrogen, $-NH_2$, $-NHCOCH=CH_2$ or a group containing vinyl or vinylene in its structure. The compound reversibly isomerizes upon being irradiated with light to thereby cause a change in wettability. An image forming device wherein a photosensitive medium containing this compound is selectively exposed and ink is selectively adhered to the surface of this medium to form an image, which is transferred and fixed.

EP 0 504 419 A1

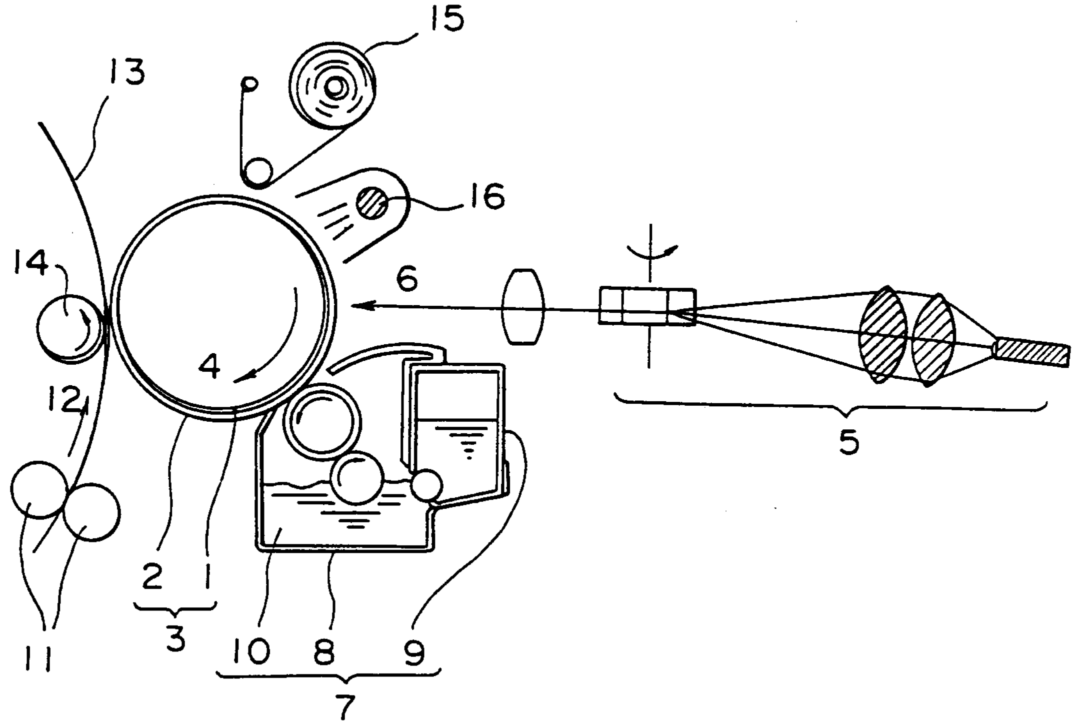


FIG. 1

## BACKGROUND OF THE INVENTION

### TECHNICAL FIELD

The present invention relates to a novel azobenzene compound. More particularly, the present invention is concerned with an azobenzene compound having a wettability changeable by a reversible isomerization through light irradiation, and a photoreceptor composition comprising the same. Further, the present invention relates to an image forming device using said compound.

### BACKGROUND ART

Japanese Patent Laid-Open Publication No. 255857/1989 disclosed an image forming device wherein an image forming material comprising a compound having a wettability changeable by a reversible isomerization through light irradiation (hereinafter referred to as a "photo reversible isomerization compound") is subjected to exposure and development to selectively adhere an ink on the surface of the image forming material and the ink image is transferred and fixed on a recording medium to prepare a print.

## SUMMARY OF THE INVENTION

An object of the present invention is to provide an azobenzene compound usable as a photoreceptor of the above image forming device and having a wettability changeable by a reversible isomerization through light irradiation, and a photoreceptor composition comprising the same.

Another object of the present invention is to provide an image forming device using said azobenzene compound.

The present invention provides a compound represented by the following general formula (I):

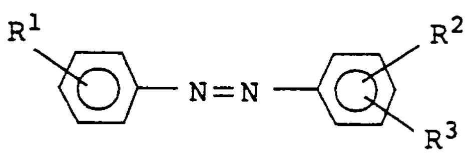

(I)

wherein $R^1$ and $R^2$ which may be the same or different each represent a halogen atom, a $C_{1-6}$ alkyl group substituted by a halogen atom, $-NO_2$, $-SC{\equiv}N$, $-C{\equiv}N$, $-SO_3H$, $-C_nF_{2n}COOF$, $-C_nF_{2n}COF$, $-C_nF_{2n}COOC_mF_{2m+1}$ - (where n and m are each an integer from l to 6), $-SO_2NH_2$, $-SO_2NHCF_3$ or $-SO_2NHCH_3$; and

$R^3$ represents a hydrogen atom, $-NH_2$, $-NHCOCH=CH_2$ or a group having therein a vinyl group or a vinylene group.

Further, the present invention provides a polymer compound having as its side chain an azo compound residue represented by the following general formula (II):

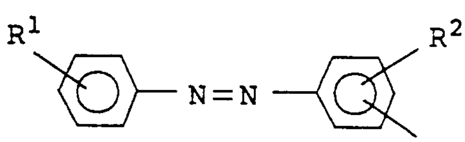

(II)

## BRIEF DESCRIPTION OF THE DRAWINGS

Fig. 1 is a diagram showing the structure of an image forming device wherein use may be made of the photoreceptor composition according to the present invention;

Fig. 2 is an infrared absorption spectrum of 2,2'-dinitro-4-aminoazobenzene (DNAA);

Fig. 3 is a diagram showing a change of an ultraviolet and visible light absorption spectrum of DNAA upon being exposed to light, wherein Fig. 3A shows the results in the case where ultraviolet light is applied and Fig. 3B shows the results in the case where the application of the ultraviolet light is stopped;

Fig. 4 is an infrared absorption spectrum of 2,2'-difluoro-4-aminoazobenzene (DFAA);

Fig. 5 is a diagram showing a change of an ultraviolet and visible light absorption spectrum of DFAA upon being exposed to light, wherein Fig. 5A shows the results in the case where ultraviolet light is applied and Fig. 5B shows the results in the case where the application of the ultraviolet light is stopped; and

Fig. 6 is a schematic diagram of a photoreceptor comprising a plurality of photo reversible isomerization compound.

DETAILED DESCRIPTION OF THE INVENTION

Azo Compound

The present invention provides a compound represented by the general formula (I).

In the general formula (I), the $R^1$ and $R^2$ which may be the same or different each represent a halogen atom (for example, F, Cl, Br or I with F being preferred), a $C_{1-6}$ alkyl group substituted by a halogen atom, $-NO_2$, $-SC{\equiv}N$, $-C{\equiv}N$, $-SO_3H$, $-C_nF_{2n}COOF$, $-C_nF_{2n}COF$, $-C_nF_{2n}COOC_mF_{2m+1}$ (where n and m are each an integer from 1 to 6), $-SO_2NH_2$, $-SO_2NHCF_3$ or $-SO_2NHCH_3$.

The $C_{1-6}$ alkyl group substituted by a halogen atom in the $R^1$ and $R^2$ is preferably a $C_{1-4}$ alkyl group and preferably substituted by at least one F.

The $R^1$ and $R^2$ are each a substituent in the benzene ring portion of the azobenzene, and the position of the substitution may be either o- or m-position to the azo group. In the $R^1$, the position of the substitution may further be p-position. In the compound according to the present invention, both the $R^1$ and $R^2$ are preferably located at the o- or m-position to the azo group.

In the general formula (I), $R^3$ represents a hydrogen atom, $-NH_2$, $-NHCOCH{=}CH_2$ or a group having therein a vinyl group or a vinylene group. Although the $R^3$ may be located at the o-, m- or p-position to the azo group, the p- or m-position is preferred. As will be described later, the compound of the general formula (I) according to the present invention may be present as a side chain of a polymer compound. In the synthesis of such a polymer compound, the $R^3$ preferably has a vinylene group therein. This is because when the $R^3$ has a vinylene group therein, it becomes possible to easily prepare a homopolymer or a copolymer through a vinyl polymerization.

The compound represented by the general formula (I) can take cis- and trans-configurations to the azo group. All the isomers and their mixtures fall within the scope of the present invention.

Further, the present invention provides a polymer compound having as its side chain an azo compound represented by the general formula (II).

In the general formula (II), the $R^1$ and $R^2$ which may be the same or different each represent a halogen atom, a $C_{1-6}$ alkyl group substituted by a halogen atom, $-NO_2$, $-SC{\equiv}N$, $-C{\equiv}N$, $-SO_3H$, $-C_nF_{2n}COOF$, $-C_nF_{2n}COF$, $-C_nF_{2n}COOC_mF_{2m+1}$ (where n and m are each an integer from 1 to 6), $-SO_2NH_2$, $-SO_2NHCF_3$ or $-SO_2NHCH_3$.

The residue represented by the general formula (II) is one formed by eliminating the substituent $R^3$ from the general formula (I). The polymer compound according to the present invention includes all the compounds wherein the residue represented by the general formula (II) is directly bonded to a main chain of the polymer compound through the substituent $R^3$ in the general formula (I) or through a group other than the group $R^3$ or without intervention of any group. A group wherein $R^3$ has a group $-NHCOCH{=}CH_2$ or a vinylidene group therein is particularly preferred from the viewpoint of easiness of the production of a polymer compound (including a homopolymer and a copolymer) by vinyl polymerization of a double bond present in these groups.

In the polymer compound represented by the general formula (II), the $R^1$ and $R^2$ are each a substituent in the benzene ring portion of an azobenzene. The position of the substituent may be either the o- or m-position to the azo group. In the $R^1$, the p-position may also be possible. Both the $R^1$ and $R^2$ are preferably located at either the o- or m-position to the azo group.

Further, the polymer compound represented by the general formula (II) as well can take cis- and transconfigurations to the azo group. All the isomers and their mixtures fall within the scope of the present invention.

There is no particular limitation on the main chain of the polymer compound according to the present invention as far as it does not inhibit the isomerization of the residue represented by the general formula (II) through light irradiation which will be described later.

According to a preferred embodiment of the present invention, a polymer compound having in its main chain a structure represented by the following general formula (III):

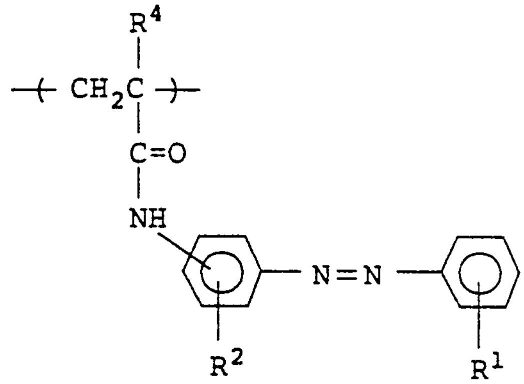

(III)

wherein $R^1$ and $R^2$ which may be the same or different each represent a halogen atom, a $C_{1-6}$ alkyl group substituted by a halogen atom, a $C_{1-6}$ alkyl group substituted by a halogen atom, $-NO_2$, -SC≡N, -C≡N, $-SO_3H$, $-C_nF_{2n}COOF$, - $C_nF_{2n}COF$, $-C_nF_{2n}COOC_mF_{2m+1}$ (where n and m are each an integer from 1 to 6), $-SO_2NH_2$, $-SO_2NHCF_3$ or $-SO_2NHCH_3$; and

$R^4$ represents a hydrogen atom, a $C_{1-6}$ alkyl group, preferably a $C_{1-4}$ alkyl group.

In the polymer compound represented by the general formula (III), at least one recurring unit should be present. When a plurality of recurring units are present, the $R^1$ and $R^2$ may be the same or different. When the $R^1$ and $R^2$ are different from each other, the arrangement of the recurring units may be in a random or block form. A polymer compound having various properties such as a proper change in the wettability can be prepared through proper control of the kind and content of the $R^1$ and $R^2$.

According to another preferred embodiment of the present invention, the polymer compound having in its main chain a structure represented by the general formula (III) is a polymer compound having in its main chain recurring units represented by the following general formula (IV):

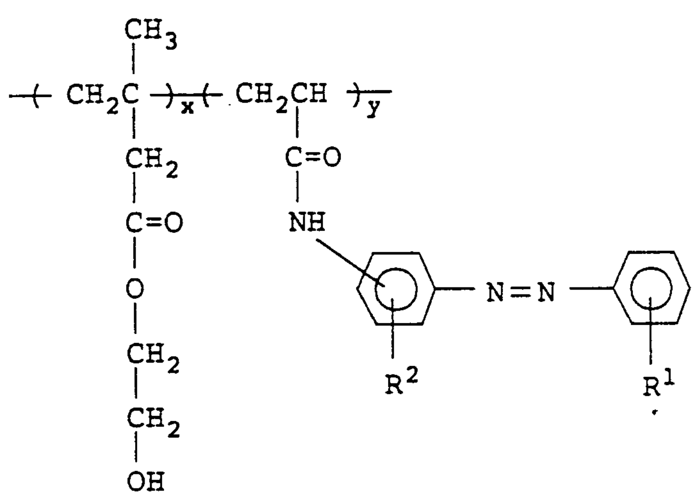

(IV)

wherein $R^1$ and $R^2$ which may be the same or different each represent a halogen atom, a $C_{1-6}$ alkyl group substituted by a halogen atom, $-NO_2$, -SC≡N, -C≡N, $-SO_3H$, $-C_nF_{2n}COOF$, $-C_nF_{2n}COF$, $-C_nF_{2n}COOC_mF_{2m+1}$ - (where n and m are each an integer from 1 to 6), $-SO_2NH_2$, $-SO_2NHCF_3$ or $-SO_2NHCH_3$; and x and y are each a natural number.

In the polymer compound represented by the general formula (IV), the x and y which represent a degree of polymerization are each a natural number of 1 or more. The x is preferably in the range of from about 50 to 700, still preferably in the range of from about 100 to 350. The y is preferably in the range of from about 50 to 500, still preferably in the range of from about 100 to 250.

As is apparent from the formula (IV), the polymer compound represented by the formula (IV) is a vinyl polymer of a compound represented by the general formula (I) wherein $R^3$ stands for $-NHCOCH=CH_2$ (this compound will be hereinafter often referred to as "the compound represented by the general formula (Ib)") with 2-hydroxyethyl methacrylate. The arrangement of each monomer in the polymer chain represented by

the general formula (Ib) may be in a random or block form. Further, as described above, the recurring units may be the same or different with respect to the $R^1$ and $R^2$.

In general, the structure of the terminal group of the polymer compound according to the present invention has no significant influence on the properties of the polymer compound as far as the molecular weight of the polymer compound is large. For example, in the process which will be described later, in terms of the probability, the terminal structure generally comprises a compound represented by the general formula (Ib) or 2-hydroxyethyl methacrylate.

According to a further preferred embodiment of the present invention, the polymer compound having in its main chain a structure represented by the general formula (III) is a high-molecular weight compound having in its main chain recurring units represented by the following general formula (V):

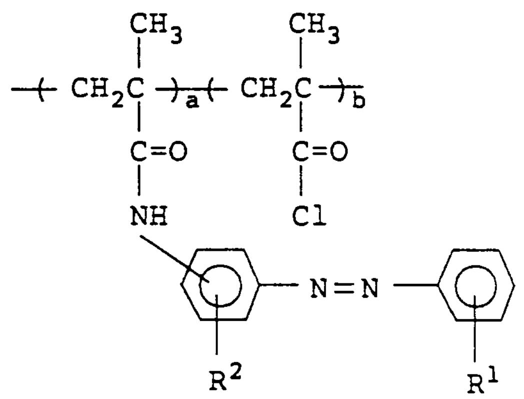

(V)

wherein $R^1$ and $R^2$ which may be the same or different each represent a halogen atom, a $C_{1-6}$ alkyl group substituted by a halogen atom, $-NO_2$, -SC≡N, -C≡N, $-SO_3H$, $-C_nF_{2n}COOF$, $-C_nF_{2n}COF$, $-C_nF_{2n}COOC_mF_{2m+1}$ - (where n and m are each an integer from 1 to 6), $-SO_2NH_2$, $-SO_2NHCF_3$ or $-SO_2NHCH_3$; and a and b are each a natural number.

In the polymer compound represented by the general formula (IV), the a and b which represent a degree of polymerization are each a natural number of 1 or more.

As is apparent from the formula (V), the polymer compound represented by the formula (V) is a compound formed by replacing a chlorine atom of polymethacrylic chloride with a compound represented by the general formula (II) through -NH-. Although there is no particular limitation on the molecular weight of the polymethacrylic chloride as the main chain, the molecular weight is preferably in the range of from 5,000 to 1,000,000, still preferably in the range of from 10,000 to 50,000, in terms of the number average molecular weight.

Specific preferred examples of the compound in the present invention include
2,2'-dinitro-4-aminoazobenzene (o-DNAA),
3,3'-dinitro-4-aminoazobenzene (m-DNAA),
3'-nitro-4'-((2-nitrophenyl)azo)acrylanilide (o-NNAA),
2'-nitro-4'-((2-nitrophenyl)azo)acrylanilide (m-NNAA),
2,2'-difluoro-4-aminoazobenzene (o-DFAA),
3,3'-difluoro-4-aminoazobenzene (m-DFAA),
3'-fluoro-4'-((2-fluorophenyl)azo)acrylanilide (o-FFAA),
2'-fluoro-4'-((2-fluorophenyl)azo)acrylanilide (m-FFAA),
2,2'-dicyano-4-aminoazobenzene (o-DCAA),
3,3'-dicyano-4-aminoazobenzene (m-DCAA),
3'-cyano-4'-((2-cyanophenyl)azo)acrylanilide (o-CCAA),
2'-cyano-4'-((2-cyanophenyl)azo)acrylanilide (m-CCAA),
2,2'-disulfo-4-aminoazobenzene (o-DSAA),
3,3'-disulfo-4-aminoazobenzene (m-DSAA),
3'-sulfo-4'-((2-sulfophenyl)azo)acrylanilide) (o-SSAA),
2'-sulfo-4'-((2-sulfophenyl)azo)acrylanilide (m-SSAA),
3-trifluoromethyl-4-(2-trifluoromethylphenylazo)aniline,
2-trifluoromethyl-4-(3-trifluoromethylphenylazo)aniline,
3'-trifluoromethyl-4'-[(2-trifluorophenyl)azo]acrylanilide, and

2'-trifluoromethyl-4'-[(3-trifluorophenyl)azo]acrylanilide.

Production of Compound

The compound and polymer compound represented by the general formulae (I) to (IV) according to the present invention can be produced by a process conforming to the purposes. A favorable example of the process will now be described.

A compound represented by the following general formula (Ia) which is a compound represented by the general formula (I) wherein $R^3$ represent a group $-NH_2$ can be produced by reacting a diazonium compound represented by the general formula (V) with a compound represented by the general formula (VI):

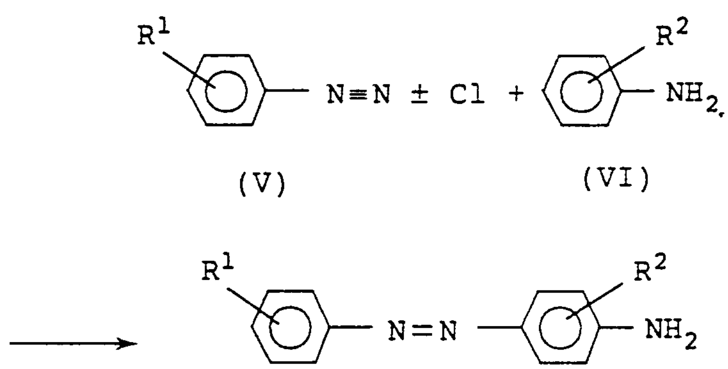

(V) (VI)

(Ia)

In the general formulae (V) and (VI), $R^1$ and $R^2$ are as defined above in connection with the general formula (I).

The reaction proceeds easily and smoothly at a temperature of about 0 to 5°C in a suitable solvent which does not participate in the reaction, for example, an alcohol such as N,N-dimethylformamide (DMF), tetrahydrofuran or methanol, or water.

The compound represented by the general formula (V) can be prepared, for example, by converting an aniline derivative substituted by $R^1$ to a hydrochloride and treating the hydrochloride with sodium nitrite.

A compound represented by the following general formula (Ib) which is a compound represented by the general formula (I) wherein $R^3$ represents a group $-NHCOCH=CH_2$ can be produced by reacting the compound represented by the general formula (Ia) with acryloyl chloride:

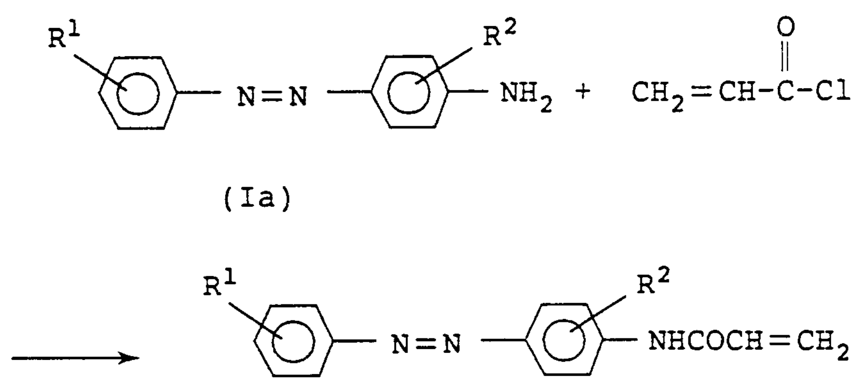

(Ia)

(Ib)

The reaction proceeds easily and smoothly at a temperature of about 10 to 50°C in a suitable solvent which does not participate in the reaction, for example, DMF, tetrahydrofuran (THF), an alcohol such as methanol, benzene or toluene.

Since the compound represented by the general formula (Ib) has a vinyl group, a homopolymer

represented by the general formula (Ib) can be prepared by vinyl polymerization, that is, by conducting the reaction under a vinyl polymerization condition.

The reaction is conducted, for example, by bringing the reactants into contact with each other at a time, stepwise or in a plurality of times through the use of a suitable initiator or light irradiation.

Further, it is also possible to prepare a copolymer by vinyl polymerization with a suitable compound having a vinyl group. There is no particular limitation on the suitable vinyl monomer as far as the monomer is vinylpolymerizable, and example thereof include acrylic and methacrylic compounds, such as acrylic acid, methacrylic acid, an alkyl acrylate, an alkyl methacrylate and a hydroxyalkyl acrylate, and styrenic and maleic compounds.

In the production of a copolymer with the above vinyl monomer, while the ratio of the compound represented by the general formula (Ib) to the other vinyl monomer is desirably 1:1, it may be varied according to the desired molecular weight or the desired terminal group. Specifically, in terms of the probability, when the ratio is 1:1, respective reactants exist at the terminal. When one reactant is used in an excess amount, the excess reactant exists at the terminal.

Further, a block polymer wherein different recurring units are arranged in a block form is prepared through the addition polymerization of a compound (Ib) different from the above compound (Ib) in the $R^1$ and $R^2$ and/or a different kind of vinyl monomer to the terminal of the once produced polymer compound. On the other hand, a random copolymer is prepared when a plurality of compounds (Ib) and/or vinyl monomers are used from the beginning.

The polymer compound represented by the general formula (IV) which is a preferred embodiment of the present invention can be prepared by reacting a compound represented by the general formula (Ib) with 2-hydroxyethyl methacrylate.

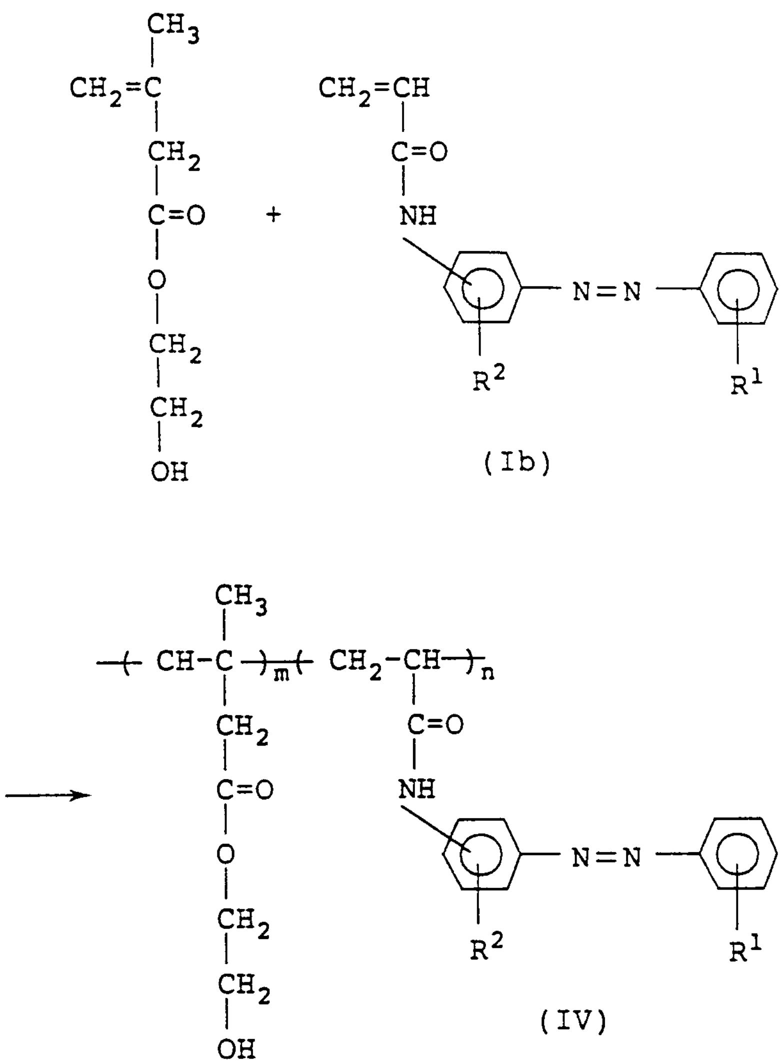

(Ib)

(IV)

wherein $R^1$ and $R^2$ are as defined above in connection with the general formula (I) and n and m are each a natural number from 1 or more.

The polymer compound represented by the general formula (V) can be prepared by reacting polymethacrylic chloride as the main chain with a compound represented by the general formula (I) wherein $R^3$ stands for $-NH_2$.

The reaction can be conducted at a temperature of about 70 to 80°C in a solvent which does not participate in the reaction (for example, toluene) preferably under a deoxygenated condition.

Applications of Compounds and Image Forming Device

The compound represented by the general formula (I) according to the present invention is reversibly isomerized with respect to the azo group upon being exposed to light having a particular wavelength. Isomerization between cis- and trans-configurations occurs with respect to the azo group. In this case, this greatly changes the wettability. When the compound represented by the general formula (I) has a trans-configuration to the azo group, this compound becomes non-polar and exhibits a good wettability by a non-polar solvent. On the other hand, when the compound has a cis-configuration to the azo group, this compound has a large polarity and exhibits a good wettability by a polar solvent. These properties are maintained in not only the compound represented by the general formula (I) but the polymer compounds represented by the general formulae (II) to (IV) according to the present invention.

Although specific wavelength of the light to be applied varies depending upon the kinds of $R^1$ and $R^2$ and the kind of the polymer compound existing as a main chain, the configuration changes to the cis-configuration by ultraviolet light irradiation, and the configuration changes to the trans-configuration by visible light irradiation. This change is reversible. For example, in the case of the above m-FFAA, the configuration becomes cis-configuration when the compound is irradiated with a light having a wavelength of 400 nm or less, while the configuration becomes trans-configuration when the compound is irradiated with a light having a wavelength of 450 nm or more.

By virtue of the above properties, the compounds represented by the general formulae (I) to (IV) and the polymer compounds according to the present invention can be utilized as a photoreceptor of an image forming device described in Japanese Patent Laid-Open publication No. 255857/1989.

The image forming device described in Japanese Patent Laid-Open Publication No. 255857/1989 will now simply be described with reference to Fig. 1.

An image forming material 3 comprising a substrate 1 and, coated thereon, a photo reversible isomerization layer (a photoreceptor) 2 is a drum in a cylindrical form which is rotatively driven in the direction of an arrow 4. The photoreceptor 2 is isomerized from the transconfiguration to the cis-configuration and vice versa through selective exposure 6 by means of an exposing device (for example, a nitrogen or semiconductor laser, a light emitting diode, a line light source and a liquid crystal shutter or the like) 5 to form a latent image on the image forming material 3. The formed latent image is visualized by means of an ink 10 when it passes through a developing device 7. Since the trans-isomer is non-polar, although a non-polar solution adheres, a solution having a large polarity does not adhere. On the other hand, since the cis-isomer has a large polarity, although a non-polar solution does not adhere, a solution having a large polarity adheres. Therefore, for example, when the change of the trans-isomer to the cis-isomer through selective exposure 6 is intended, the ink may be used in the form of a polar solution for the purpose of forming a negative image and in the form of a non-polar solution for the purpose of forming a positive image. The developing device 7 comprises an ink feeding device 8 and an ink cartridge 9. The ink 10 is fed on the surface of the image forming material 3 after the thickness of the ink is adjusted by a plurality of rollers and the surface and the roughness of the roller. The image forming material 3 on which an ink image has been formed is fed in the direction of an arrow 12 by means of a transfer roller 11. The image forming material comes into contact with a recording medium 13 which moves in synchronism with the image forming material 3, and the ink image is transferred from the image forming material 3 to the recording medium 13 and fixed by means of a pressure fixation device 14. The ink remaining untransferred on the surface of the image forming material 3 wherein the transfer has been completed is removed by means of a cleaning device 15. The image forming material 3 is returned to the cis- or trans-isomer through light irradiation by means of an erase lamp 16, and the subsequent step of forming an image is conducted.

When the use as a photoreceptor of the image forming device is intended, a dispersion of the compound represented by the general formula (I) according to the present invention in a suitable carrier or a solvent or a dispersion of the polymer compound represented by the general formulae (II) to (IV) in a suitable carrier or a solvent is applied as a photoreceptor on the image forming material 3 to form a photo reversible isomerization layer (a photoreceptor) 2.

Specifically, the compound represented by the general formula (I) is dissolved or dispersed in an organic solvent as a solvent or a carrier, for example, an alcohol such as methanol or ethanol, an ether such as THF or ethyl ether, or an amide such as DMF, and the solution or dispersion is coated or sprayed on a support 1 to form a photoreceptor. In the case of the polymer compounds represented by the general formulae (II) to (IV), a photoreceptor can be formed in the same manner as that in the case of the compound represented by the general formula (I) through the use of THF, chloroform, DMF or the like as a solvent or a carrier. The thickness of the photoreceptor is preferably in the range of from about 1 to 500 $\mu$m.

A photosensitizer, such as an organonickel complex, a hindered amine compound or a bis-sebacate compound, or a trigger material for a reversible or irreversible chain reaction may be incorporated in the photoreceptor.

According to a further embodiment of the present invention, in the above image forming device, a plurality of photo reversible isomerization compounds are preferably present in the photoreceptor 2. When a plurality of photo reversible isomerization compounds are present in the photoreceptor, the wettability of the surface of the photoreceptor can be advantageously continuously regulated in a wide range by regulating the time of irradiation of the photoreceptor with light, the ratio of presence of different photo reversible isomerization compounds in the photoreceptor and a combination thereof. When the change in the contact angle is large, the change in the contact angle best suitable for the intended ink can be selected. Consequently, the transfer and the fixation state of the ink can be improved, and the amount of a residual ink on the photoreceptor can be advantageously reduced. The isomerization of a plurality of photo reversible isomerization compounds may be conducted at the same wavelength or a different wavelength. The compounds represented by the general formulae (I) to (V) may be selected as one of the plurality of photo reversible isomerization compounds. Further examples of the photo reversible isomerization compound include stilbene, spiropyran, spirooxazine, fulgide, nitrobenzyl, triphenylmethanol, indigo, dihydroprene and anil compounds.

Although there is no particular limitation on the form of presence of these plurality of photo reversible isomerization compounds in the photoreceptor, the presence in the polymer chain or as a side chain in the polymer chain is preferred because the isomerization can be stably conducted. According to the first preferred embodiment of the present invention, as shown in Fig. 6A, a plurality of photo reversible isomerization compounds are allowed to exist as a side chain in an identical polymer chain. According to the second preferred embodiment of the present invention, as shown in Fig. 6B, a plurality of photo reversible isomerization compounds are allowed to exist as a side chain in respective different polymer chains. The polymer compound according to this embodiment is preferred from the viewpoint of easiness of the synthesis as compared with the polymer compound according to the first embodiment. According to the third preferred embodiment of the present invention, as shown in Fig. 6C, a plurality of photo reversible isomerization compounds are allowed to exist in the main chain in an identical polymer chain. This is because in some compounds, the introduction of the compound in the main chain provides a more stable photo isomerization than the introduction of the compound as a side chain. Further, according to the fourth preferred embodiment of the present invention, as shown in Fig. 6D, a plurality of photo reversible isomerization compounds are allowed to exist in the main chain in respective different polymer chains. The polymer compound according to this embodiment is preferred from the viewpoint of easiness of synthesis as compared with the polymer compound according to the third embodiment of the present invention.

## EXAMPLES

### Example 1:

#### 2,2'-Dinitro-4-aminoazobenzene (o-DNAA)

(1) 2-Nitrobenzenediazonium hydrochloride

Water (10 ml) and 35% hydrochloric acid (12.5 g) were added to 98% o-nitroaniline (5.638 g) previously dissolved in 150 g of DMF, and the mixture was vigorously stirred to give an ammonium salt solution.

The ammonium salt solution was cooled at 0 to 5°C, and a solution of sodium nitrite dissolved in water (1 ml) of 3°C was dropwise added thereto so that the temperature of the reaction mixture does not become 5°C or above. After the completion of the dropwise addition, the stirring of the reaction mixture was continued for one hour while maintaining the temperature at 5°C or below.

(2) o-DNAA

98% m-nitroaniline (5.638 g) was dissolved in DMF (200 ml). The solution was cooled to 5 ° C with iced water and then dropwise added to the solution prepared in the above step (1) so that the temperature of the reaction mixture does not become 5 ° C or above. After the completion of the dropwise addition, the reaction mixture was stirred at 0 to 5 ° C for one hour and then stirred at room temperature for 100 min to complete the reaction.

After the completion of the reaction, the reaction mixture was poured into a large amount of iced water to give a crude crystal. The crude crystal was purified through recrystallization from a solution comprising ethanol and water in a ratio of 1:1. The purified product was dried in vacuo to give 7.732 g of the title compound (yield 67.4%, melting point 167 ° C).

| Elementary analysis: | C | H | N |
|---|---|---|---|
| Calculated ($C_{12}H_9N_5$)<br>Found | 50.2<br>51.3 | 3.2<br>3.3 | 24.4<br>24.0 |

Example 2:

3'-Nitro-4'((2-nitrophenyl)azo)acrylanilide (o-NNAA)

o-DNAA (7.00 g) prepared in Example 1 was dissolved in DMF (100 ml), and a mixture of 98% acryloyl chloride (3.38 g) with DMF (15 ml) was dropwise added at 20 ° C to the resultant solution. After the completion of the dropwise addition, the mixture was stirred at 20 ° C for one hour. Further, the stirring was continued at 50 ° C for 5 hr to complete the reaction.

After the completion of the reaction, the reaction mixture was poured into a large amount of iced water to give a crude crystal product. The crude crystal product was purified through recrystallization from ethanol, and the purified product was dried in vacuo to give 5.66 g of the title compound (yield 68.5%, melting point 147 ° C).

| Elementary analysis: | C | H | N |
|---|---|---|---|
| Calculated ($C_{15}H_{11}N_5$)<br>Found | 52.8<br>53.5 | 3.3<br>3.3 | 20.5<br>20.1 |

Example 3:

3,3'-Dinitro-4-aminoazobenzene (m-DNAA)

5.21 g of the title compound was prepared in the same manner as that of Example 1, except that m-nitroaniline and o-nitroaniline were used instead of o-nitroaniline used in Example 1(1) and m-nitroaniline used in Example 1(2), respectively (yield 63.1%, melting point 178 ° C).

| Elementary analysis: | C | H | N |
|---|---|---|---|
| Calculated ($C_{12}H_9N_5$)<br>Found | 50.2<br>51.0 | 3.2<br>3.2 | 24.4<br>24.1 |

Example 4:

2'-Nitro-4'-((3-nitrophenyl)azo)acrylanilide (m-NNAA

3.12 g of the title compound was prepared in the same manner as that of Example 2, except that use was made of m-DNAA prepared in Example 3 (yield 48.0%).

| Elementary analysis: | C | H | N |
|---|---|---|---|
| Calculated ($C_{15}H_{11}N_5$) | 52.8 | 3.3 | 20.5 |
| Found | 53.0 | 3.3 | 21.1 |

Example 5: (p-NNHE)

NNAA (5.00 g = 0.015 mol) prepared in Example 2 and 2-hydroxyethyl methacrylate (HEMA, 1.19 g = 0.015 mol) were dissolved in DMF, and the solution was poured together with a polymerization initiator ($\alpha$,$\alpha$'-azobisisobutyronitrile) into a polymerization tube. The polymerization tube was mounted on a vacuum line, and the solution was solidified by liquid nitrogen and vacuum-deaerated to $10^{-5}$ Torr. The solidified sample was dissolved, and residual air was removed. The solution was again solidified and vacuum-deaerated. The above procedure was repeated five times, and the polymerization tube was allowed to stand for 72 hr in an explosionproof drier of 65°C to conduct polymerization. After the completion of the polymerization, a sample was poured into a large amount of ethyl ether to precipitate a NNAA/HEMA copolymer (p-NNHE). The solution was filtered to isolate p-NNHE, and the polymer was washed three times with ether to pure p-NNHE.

Example 6:

3-Trifluoromethyl-4-(2-trifluoromethylphenylazo)aniline (o-FFPI)

(1) 2-Trifluoromethylbenzenediazonium hydrochloride

Water (25 ml) and 35% hydrochloric acid (31.25 g) were added to 98% o-aminobenzotrifluoride (16.44 g) previously dissolved in 350 ml of DMF, and the mixture was vigorously stirred to give an ammonium salt solution.

The ammonium salt solution was cooled at 0 to 5°C, and a solution of sodium nitrite dissolved in water (5 ml) of 3°C was dropwise added thereto so that the temperature of the reaction mixture does not become 5°C or above. After the completion of the dropwise addition, the stirring of the reaction mixture was continued for one hour while maintaining the temperature at 5°C or below.

(2) o-FFPI

99% m-aminobenzotrifluoride (16.28 g) was dissolved in DMF (500 ml). The solution was cooled to 5°C with iced water and then dropwise added to the solution prepared in the above step (1) so that the temperature of the reaction mixture does not become 5°C or above. After the completion of the dropwise addition, the reaction mixture was stirred at 5°C or below for 100 min to complete the reaction.

After the completion of the reaction, the solvent was removed by vacuum distillation, and the resultant crude o-FFPI was purified through recrystallization from ethanol, and the purified product was dried in vacuo to give 19.25 g of the title compound (yield 57.7%).

| Elementary analysis: | C | H | N. |
|---|---|---|---|
| Calculated ($C_{14}H_9N_3$) | 50.46 | 2.72 | 12.61 |
| Found | 50.98 | 2.75 | 12.38 |

Example 7:

3-Trifluoromethyl-4'-[(2-trifluoromethylphenyl)azo)acrylanilide (o-FFAA)

o-FFPI (20.00 g) prepared in Example 6 was dissolved in DMF (315 ml), and 95% acryloyl chloride (6.86 g) was dropwise added at 20°C to the resultant solution. After the completion of the dropwise addition, the mixture was stirred at 20°C for one hour. Further, the stirring was continued at 50°C for 5 hr to complete the reaction.

After the completion of the reaction, the solvent was removed by vacuum distillation, and the resultant crude o-FFAA was purified through recrystallization from ethanol, and the purified product was dried in vacuo to give the title compound (yield 51.2%).

| Elementary analysis: | C | H | N |
|---|---|---|---|
| Calculated ($C_{17}H_{11}N_3F_6$)<br>Found | 54.99<br>55.12 | 2.99<br>3.08 | 11.32<br>11.30 |

Example 8:

2-Trifluoromethyl-4-(3-trifluoromethylphenylazo)aniline (m-FFPI)

20.5 g of the title compound was prepared in the same manner as that of Example 6, except that m-aminobenzotrifluoride and o-aminobenzotrifluoride were used instead of o-aminobenzotrifluoride used in Example 6(1) and m-aminobenzotrifluoride used in Example 6(2), respectively.

| Elementary analysis: | C | H | N |
|---|---|---|---|
| Calculated ($C_{14}H_9N_3F_6$)<br>Found | 50.46<br>50.77 | 2.72<br>2.83 | 12.61<br>12.41 |

Example 9:

2'-Trifluoromethyl-4'-[(3-trifluorophenyl)azo)acrylanilide (m-FFAA)

m-FFPI (20.00 g) prepared in Example 8 was dissolved in DMF (315 ml), and 95% acryloyl chloride (6.86 g) was dropwise added at 20 °C to the resultant solution. After the completion of the dropwise addition, the mixture was stirred at 20 °C for one hour. Further, the stirring was continued at 50 °C for 5 hr to complete the reaction.

After the completion of the reaction, the solvent was removed by vacuum distillation, and the resultant crude m-FFAA was purified through recrystallization from ethanol, and the purified product was dried in vacuo to give 12.6 g of the title compound (yield 56.8%).

| Elementary analysis: | C | H | N |
|---|---|---|---|
| Calculated ($C_{17}H_{11}F_6N_3$)<br>Found | 54.99<br>55.22 | 2.99<br>3.05 | 11.32<br>11.29 |

Example 10:

m-FFAA/2-hydroxyethyl methacrylate copolymer

m-FFAA (5.0 g) prepared in Example 7 was reacted with 2-ethylhexyl methacrylate (1.19 g) in the same manner as that of Example 5 to give the title compound.

The resultant copolymer was molded into a film, and the contact angle of the film to the water was measured. As a result, it was found that the contact angle which was 75° was changed to 42° after ultraviolet light irradiation.

Example 11: Synthesis of DNAA (part 2)

o-Nitroaniline (20.0 g) was dissolved in methanol (300 ml), and 15% hydrochloric acid (60 ml) was added to the solution. The solution was maintained at 0 to 5 °C, and isoamyl nitrite (40 ml) was dropwise added thereto over a period of about 10 min. After the completion of the dropwise addition, the solution was

maintained at 0 to 5°C, and the stirring was continued for additional 120 min. A solution of m-nitroaniline (20 g) in methanol (250 ml) was dropwise added thereto, and the stirring was continued for several hours. The resultant precipitate was collected by filtration and then washed with water.

A compound having an absorption around 370 nm was fractionated by liquid chromatography from the precipitate collected by the filtration.

A peak having a molecular weight of 287 was confirmed by GCMS analysis and DI method. The infrared absorption spectrum of the synthesized compound was as shown in Fig. 2.

A methanol solution of the synthesized compound was prepared, and subjected to measurement of an ultraviolet and visible light absorption spectrum of the solution. As a result, an absorption peak around 370 nm became small by ultraviolet light irradiation, and when the ultraviolet light irradiation was stopped, the size of the peak was returned to that before the irradiation (see Figs. 3A and B). Such a change is characteristic of the cis/trans photo isomerization of the azobenzene compound, and the presence of an azo bond in the synthesized compound can be confirmed.

Example 12:

4-Amino-2,2'-difluoroazobenzene (DFAA)

o-Fluoroaniline (11.1 g) was dissolved in 2 N hydrochloric acid (150 ml) and cooled (-2 to -2.5°C), and a solution of sodium nitrite (7.18 g) in water (30 ml) was dropwise added thereto over a period of 45 min. 40 min after the completion of the dropwise addition, a solution of m-fluoroaniline (11.1 g) in 2 N hydrochloric acid (50 ml) was added thereto. After stirring for 10 min, the pH was adjusted to 3 with an aqueous sodium carbonate solution. Further, the pH was adjusted to 6 by adding sodium carbonate over a period of 1.5 hr. The reaction mixture was allowed to stand for two days, and the precipitated solid was collected by filtration, washed with 1 N hydrochloric acid, water and an aqueous sodium carbonate solution and water in that order and dried to give 18.67 g of a brown powder. The brown powder was purified by silica gel chromatography (n-hexane:ethyl acetate = 5:1 to 4:1) and recrystallized twice from cyclohexane to give DFAA in a brown acicular crystal form (yield: 4.9%).

| Elementary analysis: | C | H | N |
|---|---|---|---|
| Calculated ($C_{12}H_9F_3N_2$) | 61.80 | 3.89 | 18.02 |
| Found | 61.89 | 3.85 | 18.19 |

A peak having a molecular weight of 233 could be confirmed by GCMS analysis and DI method. The infrared absorption spectrum of the synthesized compound was as shown in Fig. 4.

The synthesized compound was subjected to an ultraviolet light absorption spectrum in the same manner as that of Example 11. As a result, the absorption peak around 395 nm became small through ultraviolet light irradiation and returned to a peak before irradiation when ultraviolet light irradiation was stopped or visible light irradiation was conducted (see Figs. 5A and 5B). Such a change is characteristic of the cis/trans photo isomerization, and the presence of the azo bond in the synthesized compound could be confirmed.

Example 13:

Polymer Introduced into DNAA Side Chain

0.45 g (0.0043 mol) of polymethacrylic chloride (number average molecular weight: 50,000) and an equimolar amount of triethylamine were dissolved in 1,4-dioxane to prepare an about 100 ml of a solution. A solution of 1.0 g of DFAA in 1,4-dioxane (20 ml) was dropwise added thereto, and the mixture was stirred for 3 hr. Then, the stirring of the mixture was continued under reflux at 70°C for one hour. The reaction mixture was allowed to stand until the temperature became room temperature. The precipitate (triethylamine) was removed by filtration, and the filtrate was poured into petroleum ether in an amount of 2 to 5 times that of the filtrate to precipitate a polymer.

The polymer was collected by filtration, and dissolved in tetrahydrofuran in an amount as small as possible. The solution was poured into petroleum ether in an amount of 2 to 5 times that of the solution to precipitate the polymer. The procedure was repeated two or three times to purify the polymer.

The resultant polymer was subjected to an infrared absorption spectrum analysis. A peak derived from

a carbonyl group of acid chloride was observed around 1790 $cm^{-1}$, and a peak derived from a carbonyl group of an acid amide bond was observed around 1680 $cm^{-1}$ which is a lower frequency than that of the peak derived from the carbonyl group of the acid chloride. This suggests that DFAA has been introduced into the side chain of the polymer. The comparison of the absorption peak around 1790 $cm^{-1}$ derived from polyacrylic chloride of the starting material with the absorption peak of the resultant polymer has revealed that about 40% of the acid chloride was reacted.

Example 14:

Polymer Introduced into DNAA Side Chain

0.36 g (0.0035 mol) of polymethacrylic chloride (number average molecular weight: 20,000) and an equimolar amount of triethylamine were dissolved in 1,4-dioxane to prepare an about 100 ml of a solution. A solution of 1.0 g of DNAA in 1,4-dioxane (20 ml) was dropwise added thereto, and the mixture was stirred for 3 hr. Then, the stirring of the mixture was continued under reflux at 70°C for one hour. The reaction mixture was allowed to stand until the temperature became room temperature. The precipitate (triethylamine) was removed by filtration, and the filtrate was poured into petroleum ether in an amount of 2 to 5 times that of the filtrate to precipitate a polymer.

The polymer was collected by filtration, and dissolved in tetrahydrofuran in an amount as small as possible. The solution was poured into petroleum ether in an amount of 2 to 5 times that of the solution to precipitate the polymer. The procedure was repeated two or three times to purify the polymer.

The resultant polymer was subjected to an infrared absorption spectrum analysis. A peak derived from a carbonyl group of acid chloride was observed around 1790 $cm^{-1}$, and a peak derived from a carbonyl group of an acid amide bond was observed around 1680 $cm^{-1}$ which is a lower frequency than that of the peak derived from the carbonyl group. This suggests that DNAA has been introduced into the side chain of the polymer. The comparison of the absorption peak around 1790 $cm^{-1}$ derived from polyacrylic chloride of the starting material with the absorption peak of the resultant polymer has revealed that about 40% of the acid chloride was reacted.

Example 15: Measurement of Contact Angle

The polymers prepared in Examples 13 and 14 were each spin-coated on a quartz glass sheet to prepare a film. Examination was conducted how the contact angle of the film to water changed before and after the ultraviolet light (350 nm ± 50 nm) and when visible light was applied after the ultraviolet right irradiation. As a result, it was observed that although the contact angle became small through ultraviolet light irradiation, it returned to the contact angle before the ultraviolet light irradiation. Further, the ultraviolet light irradiation and the visible light irradiation were repeated, so that a reversible change in the contact change was maintained. A specific change in the contact angle were as shown in Table 1.

Table 1

| Polymer | Before ultraviolet light irradiation (after visible light irradiation) | After ultraviolet light irradiation |
|---|---|---|
| Ex. 13 | 86.2° | 75.3° |
| Ex. 14 | 85.9° | 70.3° |

Comparative Example

Amino azobenzene was introduced as a side chain into polymethacrylic chloride in the same manner as that of Example 13 or 14.

The polymer thus prepared was subjected to measurement of the contact angle in the same manner as that of Example 15. The results were as shown in Table 2.

Table 2

| Before ultraviolet light irradiation (after visible light irradiation) | After ultraviolet light irradiation |
|---|---|
| 80.2° | 75.4° |

## Claims

1. A compound represented by the following general formula (I):

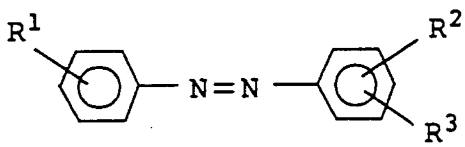

(I)

wherein $R^1$ and $R^2$ which may be the same or different each represent a halogen atom, a $C_{1-6}$ alkyl group substituted by a halogen atom, $-NO_2$, -SC≡N, -C≡N, $-SO_3H$, $-C_nF_{2n}COOF$, $-C_nF_{2n}COF$, $-C_nF_{2n}COOC_mF_{2m+1}$ (where n and m are each an integer from 1 to 6), $-SO_2NH_2$, $-SO_2NHCF_3$ or $-SO_2NHCH_3$; and
$R^3$ represents a hydrogen atom, $-NH_2$, $-NHCOCH=CH_2$ or a group having therein a vinyl group or a vinylene group.

2. A compound according to claim 1, wherein both $R^1$ and $R^2$ are located at the o- or m-position to the azo group.

3. A compound according to claim 1, wherein $R^3$ is located at the p- or m-position to the azo group.

4. A polymer compound having as its side chain an azo compound residue represented by the following general formula (II):

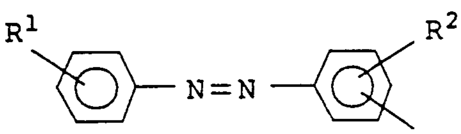

(II)

wherein $R^1$ and $R^2$ which may be the same or different each represent a halogen atom, a $C_{1-6}$ alkyl group substituted by a halogen atom, $-NO_2$, -SC≡N, -C≡N, $-SO_3H$, $-C_nF_{2n}COOF$, $-C_nF_{2n}COF$, $-C_nF_{2n}COOC_mF_{2m+1}$ (where n and m are each an integer from 1 to 6), $-SO_2NH_2$, $-SO_2NHCF_3$ or $-SO_2NHCH_3$.

5. A polymer compound having in its main chain a compound represented by the following general formula (III):

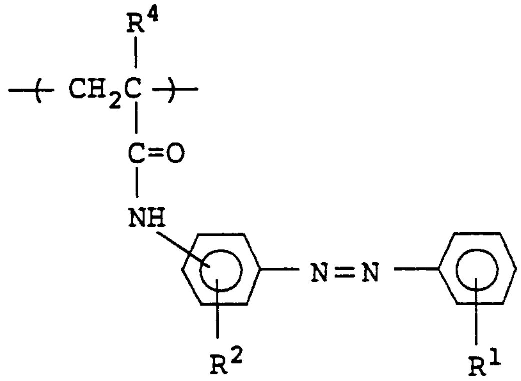

(III)

wherein $R^1$ and $R^2$ which may be the same or different each represent a halogen atom, a $C_{1-6}$ alkyl group substituted by a halogen atom, $-NO_2$, -SC≡N, -C≡N, $-SO_3H$, $-C_nF_{2n}COOF$, $-C_nF_{2n}COF$, $-C_nF_{2n}COOC_mF_{2m+1}$ (where n and m are each an integer from 1 to 6), $-SO_2NH_2$, $-SO_2NHCF_3$ or $-SO_2NHCH_3$; and $R^4$ represents a hydrogen atom or a $C_{1-6}$ alkyl group.

**6.** A polymer compound according to any one of claims 3 to 5, wherein both $R^1$ and $R^2$ are located at the o- or m-position to the azo group.

**7.** A photoreceptor composition, comprising a polymer compound according to any one of claims 1 to 6.

**8.** An image forming device comprising an image forming material comprised of a polymer compound having in its molecule a plurality of photo reversible isomerization substances; exposure means for selectively exposing said image forming material to change the wettability of the surface of said image forming material; and coating means for coating an ink from an ink accommodation portion on said image forming material; and means for transferring said ink on a recording medium.

**9.** An image forming device according to claim 8, wherein one of the photo reversible isomerization substances is an azo compound residue defined by the general formula (II) according to claim 4.

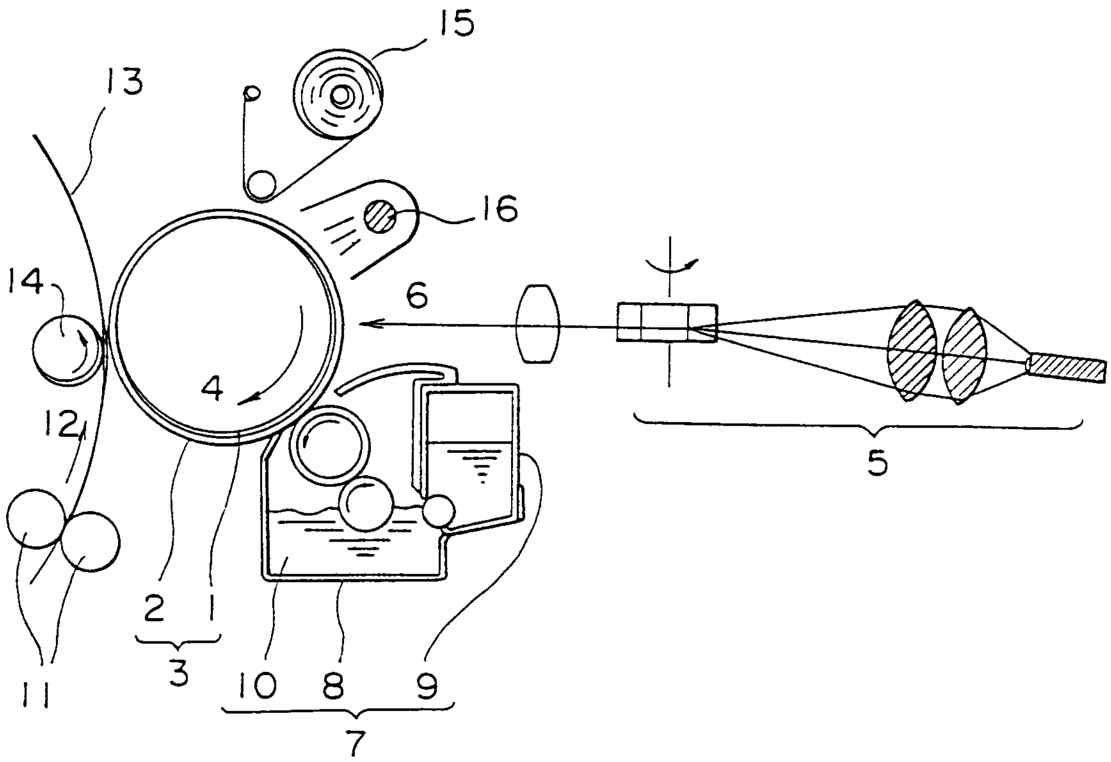


FIG. 1

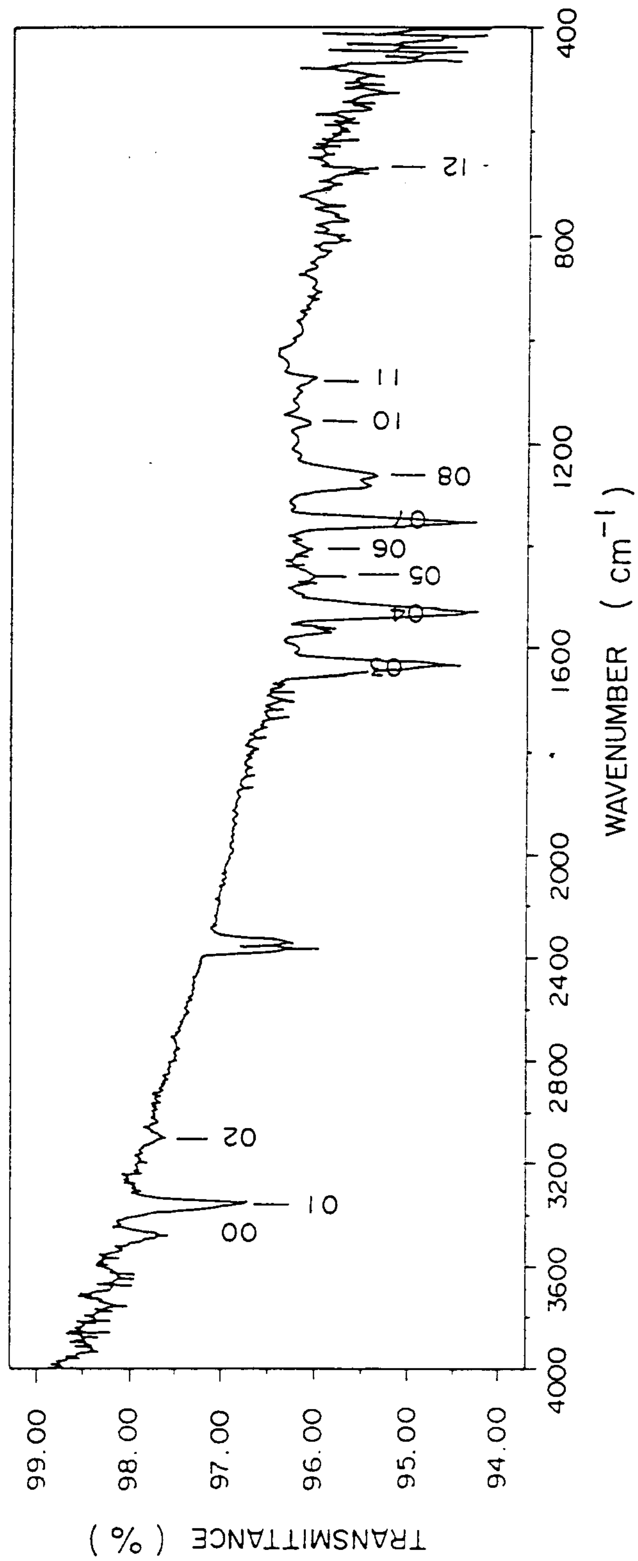
TRANSMITTANCE ( % )
99.00
98.00
97.00
96.00
95.00
94.00
WAVENUMBER ( cm$^{-1}$ )
4000
3600
3200
2800
2400
2000
1600
1200
800
400
00
01
02
03
04
05
06
07
08
10
11
12

FIG. 2

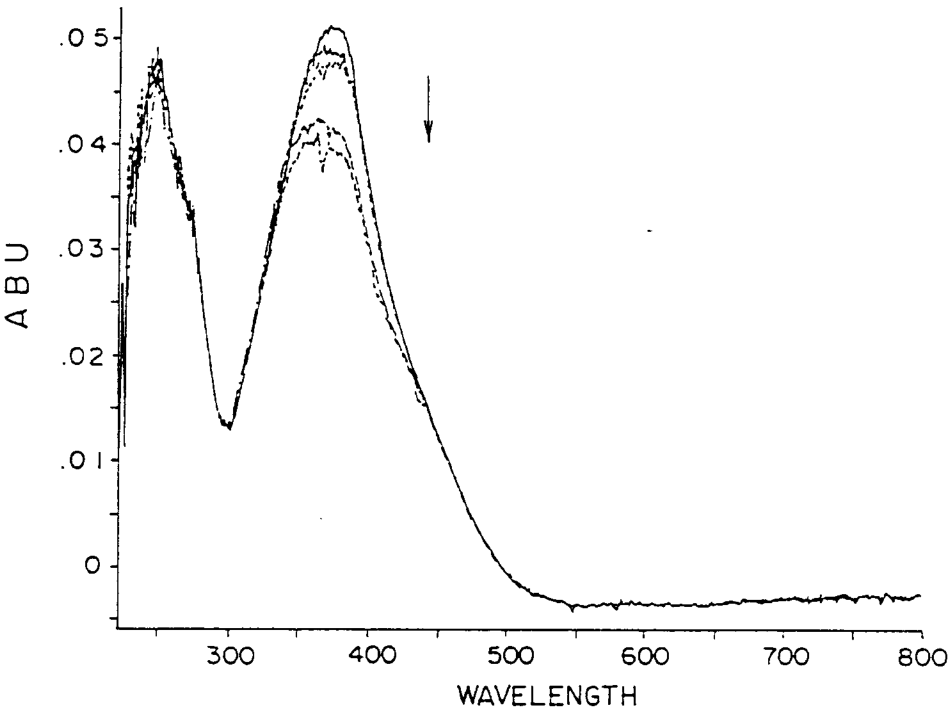
.05
.04
.03
.02
.01
0
ABU
300
400
500
600
700
800
WAVELENGTH

FIG. 3A

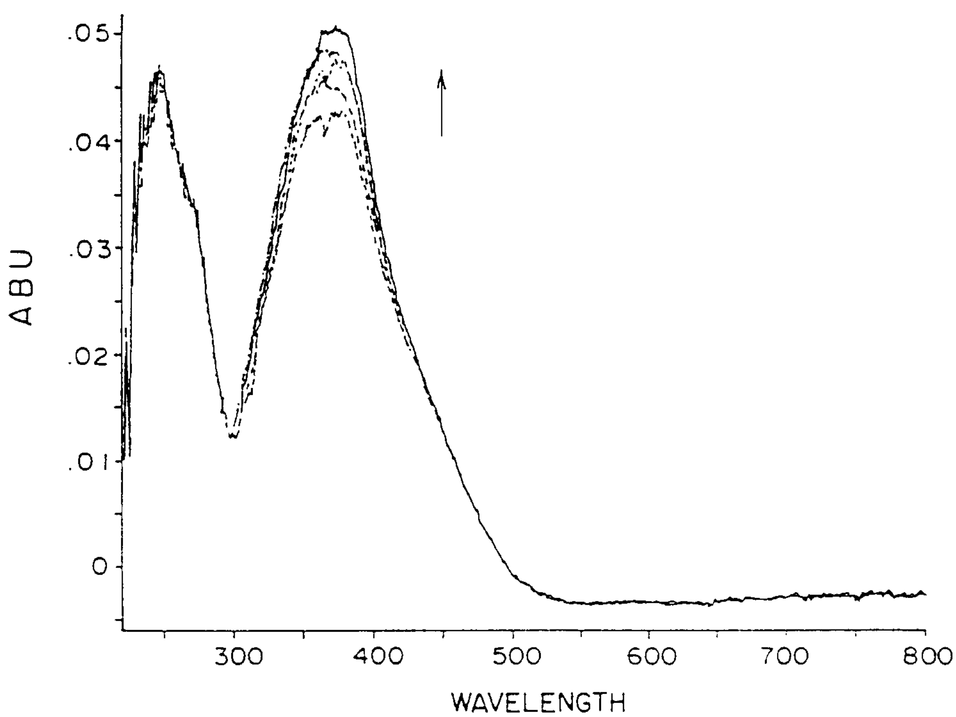
.05
.04
.03
.02
.01
0
ABU
300
400
500
600
700
800
WAVELENGTH

FIG. 3B

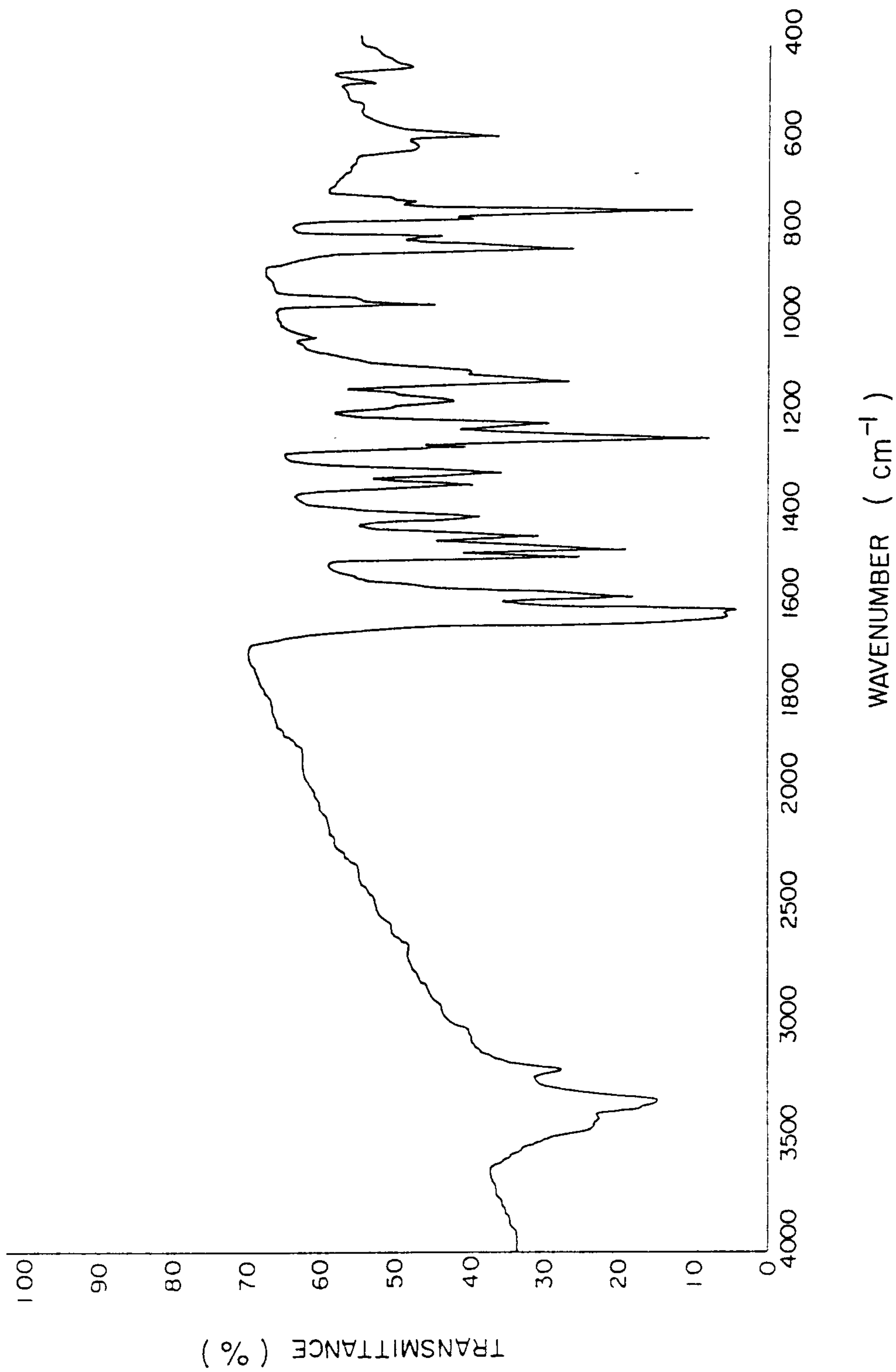
TRANSMITTANCE ( % )
100
90
80
70
60
50
40
30
20
10
0
4000
3500
3000
2500
2000
1800
1600
1400
1200
1000
800
600
400
WAVENUMBER ( $cm^{-1}$ )

FIG. 4

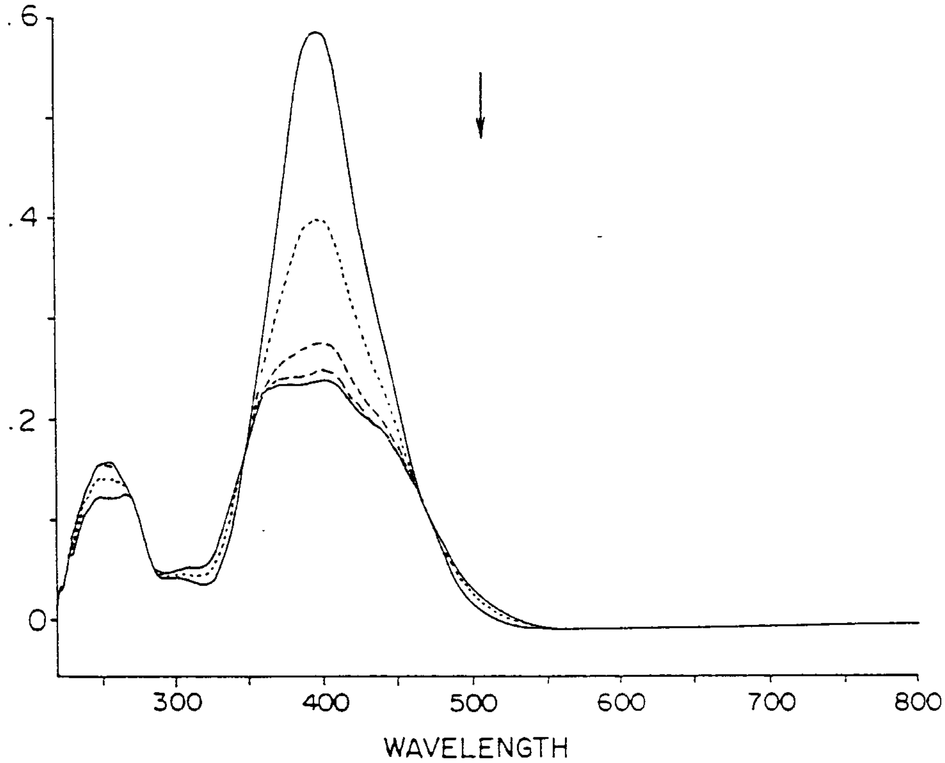
.6
.4
.2
0
300
400
500
600
700
800
WAVELENGTH

FIG. 5A

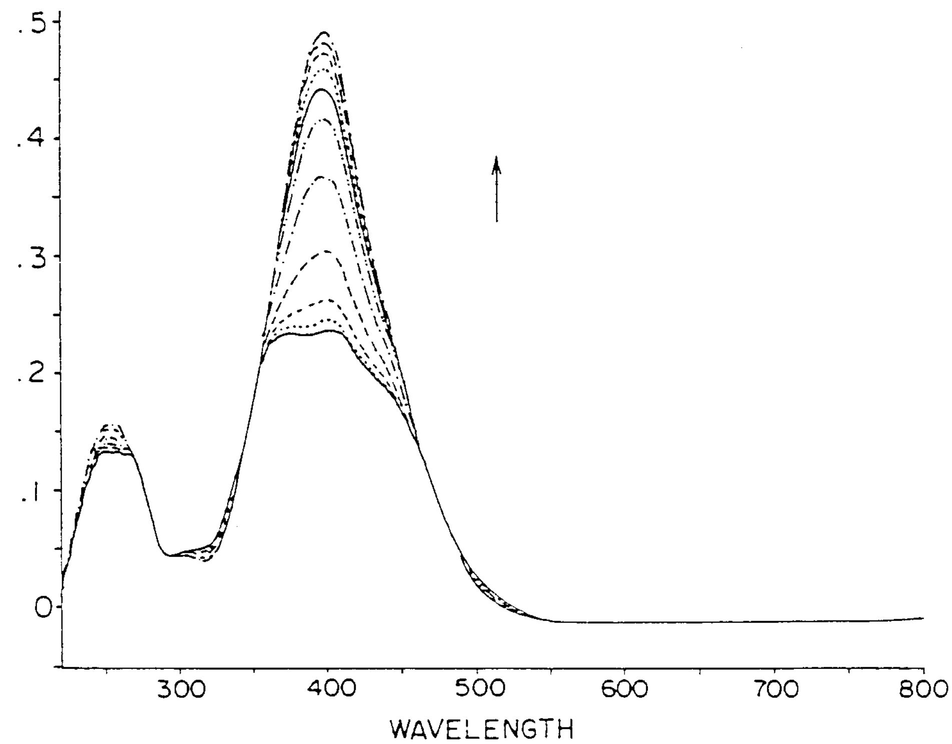
.5
.4
.3
.2
.1
0
300
400
500
600
700
800
WAVELENGTH

FIG. 5B

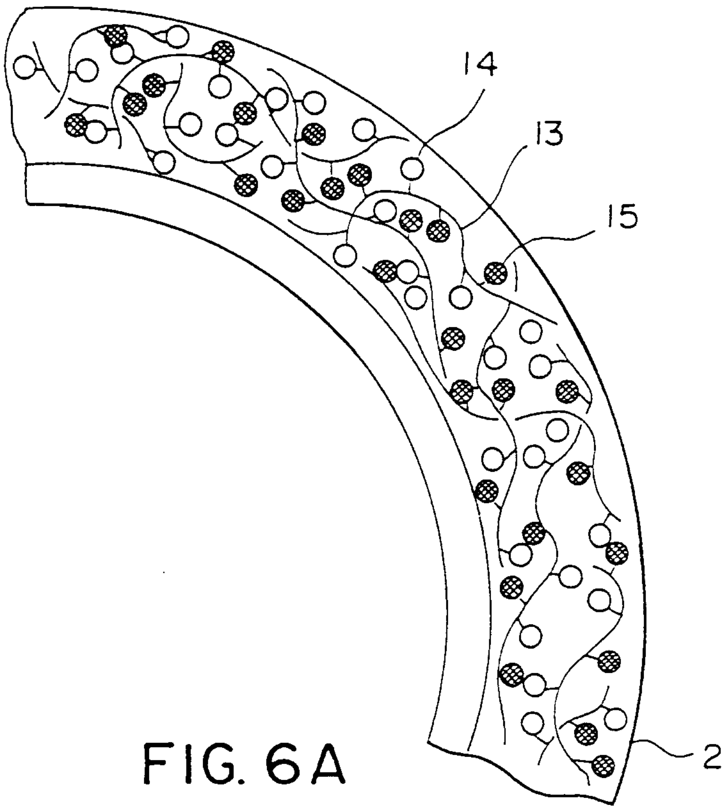


FIG. 6A

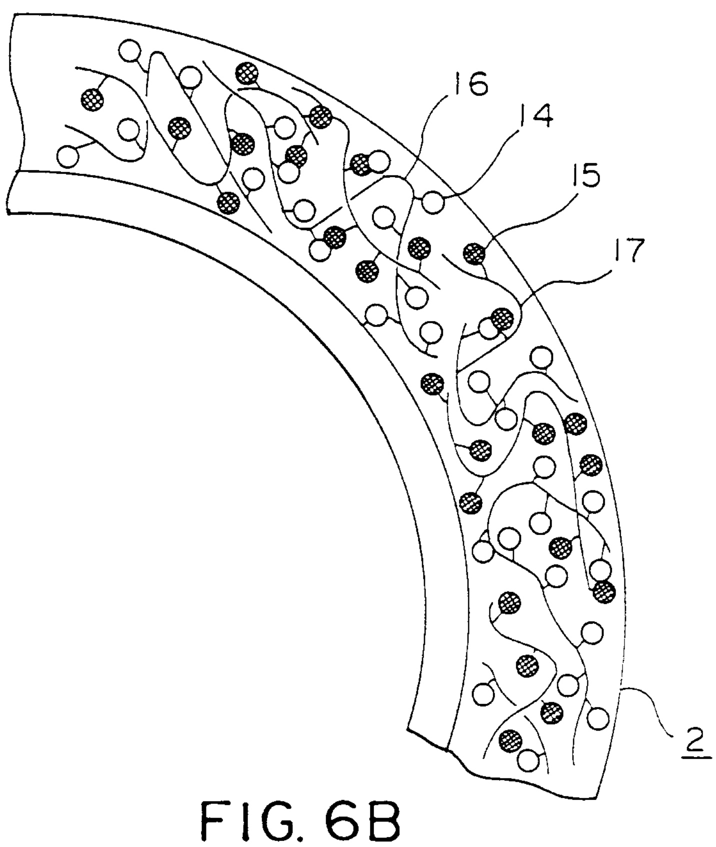


FIG. 6B

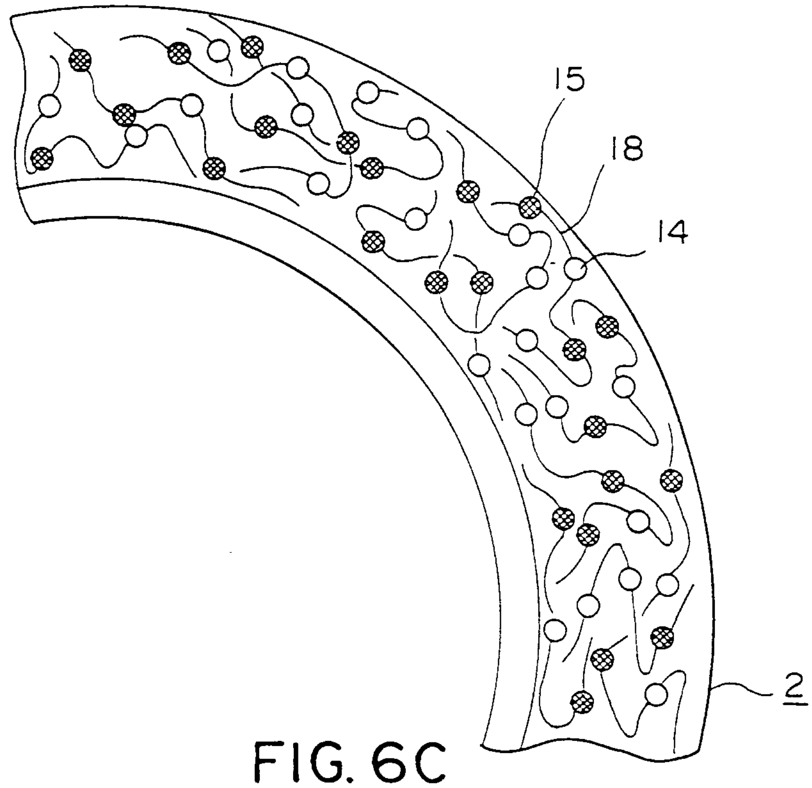


FIG. 6C

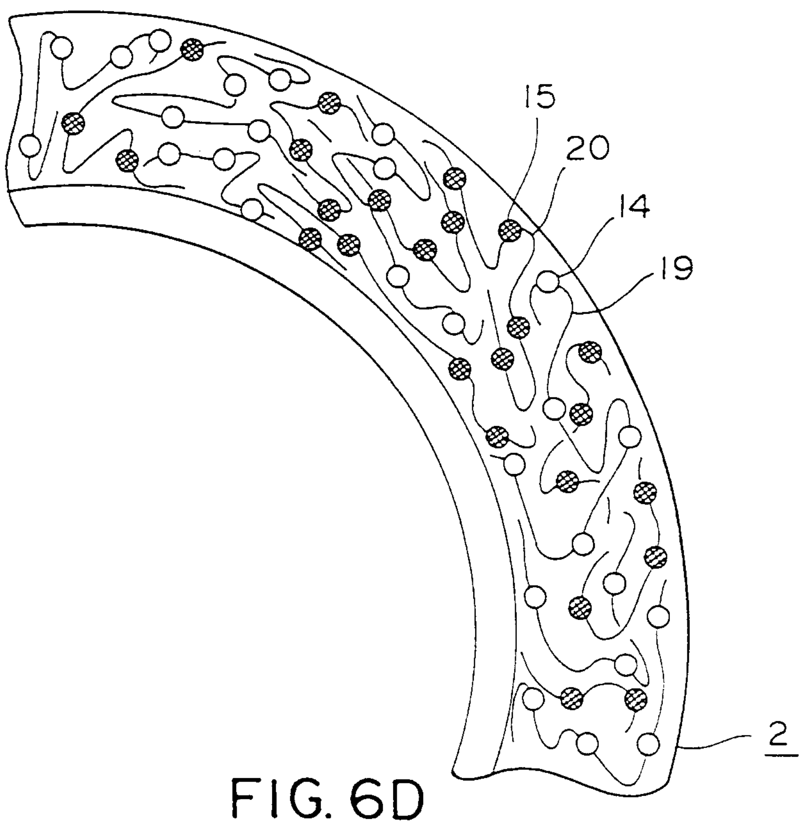


FIG. 6D

# INTERNATIONAL SEARCH REPORT

International Application No PCT/JP91/01352

**I. CLASSIFICATION OF SUBJECT MATTER** (if several classification symbols apply, indicate all) [6]

According to International Patent Classification (IPC) or to both National Classification and IPC Int. $Cl^5$

C07C245/08, 255/65, 309/46, 331/08, C08F20/60, C08F220/28, C09B69/10, C09K9/02, G03C1/73, G03F7/00

**II. FIELDS SEARCHED**

Minimum Documentation Searched [7]

| Classification System | Classification Symbols |
|---|---|
| IPC | C07C245/00, 255/00, 309/00, 331/00, C08F20/00, C08F220/00, C09B69/00, C09K9/00, G03C1/00, G03F7/00 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X,Y | JP, A, 59-89654 (The Dow Chemical Co.), May 23, 1984 (23. 05. 84), Pages 4, 7 & EP, A, 109172 | 1-9 |
| X,Y | JP, A, 53-134826 (BASF AG), November 24, 1978 (24. 11. 78), Pages 3 to 4 & DE, A, 2718620 | 1-9 |
| X,Y | JP, A, 60-41650 (Merck Patent Gesellschaft), March 5, 1985 (05. 03. 85), Pages 2 to 3 & EP, A, 134446 | 1-9 |
| X,Y | J. Chem. Soc. C, Vol. 4 (1968) Tetsuji Kametani et al. "Abnormal products from phenolic oxidation of a dihydroxy-1-benzyl-1,2,3,4-tetrahydroisoquinoline" pp. 447-451 | 1-9 |
| Y | JP, A, 61-171463 (Shin-Etsu Chemical Co., Ltd.), August 2, 1986 (02. 08. 86), Pages 1 to 2 (Family: none) | 1-9 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| December 10, 1991 (10. 12. 91) | December 24, 1991 (24. 12. 91) |
| **International Searching Authority** | **Signature of Authorized Officer** |
| Japanese Patent Office | |